# EUROPEAN PATENT SPECIFICATION

(11) **EP 1 836 215 B1**
(45) Date of publication and mention of the grant of the patent: **20.08.2008**
(21) Application number: 06700486.1
(22) Date of filing: 09.01.2006
(51) Int. Cl.: C07J 41/00, C07J 3/00, A61P 5/44, A61P 37/00, A61K 31/56

(54) **ANDROSTANE 17-ALPHA-CARBONATE FOR USE IN THE TREATMENT OF INFLAMMATORY AND ALLERGIC CONDITIONS**
ANDROSTAN-17-ALPHA-CARBONAT ZUR VERWENDUNG BEI DER BEHANDLUNG ENTZÜNDLICHER UND ALLERGISCHER ZUSTÄNDE
ANDROSTANE 17-ALPHA-CARBONATE POUR UTILISATION DANS LE TRAITEMENT DES PROBLEMES INFLAMMATOIRES ET ALLERGIQUES

(30) Priority: 10.01.2005 GB 0500406; 14.09.2005 GB 0518775
(43) Date of publication of application: 26.09.2007
(73) Proprietor: GLAXO GROUP LIMITED, Greenford, Middlesex UB6 ONN (GB)
(72) Inventor: BIGGADIKE, Keith, GlaxoSmithKline, Stevenage Hertfordshire SG1 2NY (GB); NEEDHAM, Deborah, GlaxoSmithKline, Stevenage Hertfordshire SG1 2NY (GB)
(74) Representative: Pritchard, Judith
(86) International application number: PCT/EP2006/000150
(87) International publication number: WO 2006/072600

(56) References cited:
- WO-A-20/05005451
- US-A- 4 996 335
- RACHWAL S ET AL: "Chemistry of loteprednol etabonate and related steroids. II. Reactions at ring C and NMR structural studies of the resulting compounds" STEROIDS 1998 UNITED STATES, vol. 63, no. 4, 1998, pages 193-201, XP004122439 ISSN: 0039-128X

## Description

The present invention relates to compounds which are glucocorticoid receptor agonists of the androstane series and to processes for their preparation. The present invention also relates to pharmaceutical formulations containing the compounds and to therapeutic uses thereof, particularly for the treatment of inflammatory and allergic conditions.

Glucocorticosteroids which have anti-inflammatory properties are known and are widely used for the treatment of inflammatory disorders or diseases such as asthma and rhinitis. Androstane 17α-carbonate compounds said to have anti-inflammatory activity are disclosed in U.S. patent 4,996,335. Drugs of Today 2000, 36(5), 313-320, discloses loteprednol etabonate for the treatment of allergic diseases of the airway. We have identified two novel androstane 17α-carbonate compounds.

Thus, according to one aspect of the invention, there is provided a compound of formula (I) 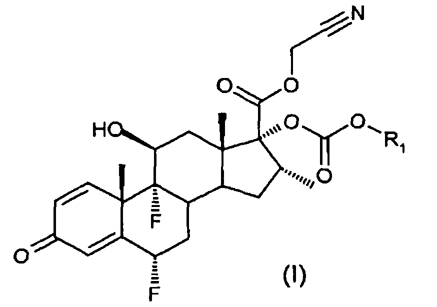 wherein
R₁ represents a 1, 1-dimethylethyl group or a 1,1-dimethylpropyl group;
or a physiologically acceptable solvate thereof.

Examples of solvates include hydrates.

References hereinafter to a compound according to the invention includes both compounds of formula (I) and solvates thereof.

One preferred compound of formula (I) is:
Cyanomethyl (6α,11β,16α,17α)-17-({[(1,1-dimethylpropyl)oxy]carbonyl}oxy)-6,9-difluoro-11-hydroxy-16-methyl-3-oxoandrosta-1,4-diene-17-carboxylate.

Another preferred compound of formula (I) is:
Cyanomethyl (6α,11β,16α,17α)-17-({[(1,1-dimethylethyl)oxy]carbonyl}oxy)-6,9-difluoro-11-hydroxy-16-methyl-3-oxoandrosta-1,4-diene-17-carboxylate.

The compounds of formula (I) have potentially beneficial anti-inflammatory or anti-allergic effects, particularly upon topical administration, demonstrated by, for example, their ability to bind to the glucocorticoid receptor and to illicit a response via that receptor. Hence, the compounds of formula (I) are potentially useful in the treatment of inflammatory and/or allergic disorders.

Examples of disease states in which the compounds of the invention may have utility include skin diseases such as eczema, psoriasis, allergic dermatitis neurodermatitis, pruritis and hypersensitivity reactions; inflammatory conditions of the nose, throat or lungs such as asthma (including allergen-induced asthmatic reactions), rhinitis (including hayfever), nasal polyps, chronic obstructive pulmonary disease, interstitial lung disease, and fibrosis; inflammatory bowel conditions such as ulcerative colitis and Crohn's disease; and auto-immune diseases such as rheumatoid arthritis.

Compounds of the invention may also have use in the treatment of conjunctiva and conjunctivitis.

It will be appreciated by those skilled in the art that reference herein to treatment extends to prophylaxis as well as the treatment of established conditions.

As mentioned above, compounds of formula (I) may be useful in human or veterinary medicine, in particular as anti-inflammatory and anti-allergic agents.

There is thus provided as a further aspect of the invention a compound of formula (I) or a physiologically acceptable solvate thereof for use in human or veterinary medicine, particularly in the treatment of patients with inflammatory and/or allergic conditions.

According to another aspect of the invention, there is provided the use of a compound of formula (I) or a physiologically acceptable solvate thereof for the manufacture of a medicament for the treatment of patients with inflammatory and/or allergic conditions.

The compounds according to the invention may be formulated for administration in any convenient way, and the invention therefore also includes within its scope pharmaceutical compositions comprising a compound of formula (I) or physiologically acceptable solvate thereof together, if desirable, in admixture with one or more physiologically acceptable diluents or carriers.

Further, there is provided a process for the preparation of such pharmaceutical compositions which comprises mixing the ingredients.

The compounds according to the invention may, for example, be formulated for oral, intranasal, buccal, sublingual, parenteral, local or rectal administration, especially local administration.

Local administration as used herein, includes administration by insufflation and inhalation. Examples of various types of preparation for local administration include ointments, lotions, creams, gels, foams, preparations for delivery by transdermal patches, powders, sprays, aerosols, capsules or cartridges for use in an inhaler or insufflator or drops (e.g. eye or nose drops), solutions/suspensions for nebulisation, suppositories, pessaries, retention enemas and chewable or suckable tablets or pellets (e.g. for the treatment of aphthous ulcers) or liposome or microencapsulation preparations.

Ointments, creams and gels, may, for example, be formulated with an aqueous or oily base with the addition of suitable thickening and/or gelling agent and/or solvents. Such bases may thus, for example, include water and/or an oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents.

Lotions may be formulated with an aqueous or oily base and will in general also contain one or more emulsifying agents, stabilising agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents, suspending agents or preservatives.

Spray compositions may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain a compound of formula (I) and a suitable propellant such as a fluorocarbon or hydrogen-containing chlorofluorocarbon or mixtures thereof, particularly hydrofluoroalkanes, especially 1,1,1,2-tetrafluoroethane, 1,1,1,2,3,3,3-heptafluoro-n-propane or a mixture thereof. The aerosol composition may optionally contain additional formulation excipients well known in the art such as surfactants e.g. oleic acid or lecithin and cosolvents e.g. ethanol.

Advantageously, the formulations of the invention may be buffered by the addition of suitable buffering agents.

Capsules and cartridges for use in an inhaler or insufflator, of for example gelatine, may be formulated containing a powder mix for inhalation of a compound of the invention and a suitable powder base such as lactose or starch. Each capsule or cartridge may generally contain between 20µg-10mg of the compound of formula (I). Alternatively, the compound of the invention may be presented without excipients such as lactose.

The proportion of the active compound of formula (I) in the local compositions according to the invention depends on the precise type of formulation to be prepared but will generally be within the range of from 0.001 to 10% by weight. Generally, however for most types of preparations advantageously the proportion used will be within the range of from 0.005 to 1% and preferably 0.01 to 0.5%. However, in powders for inhalation or insufflation the proportion used will be within the range of from 0.1 to 5%.

Aerosol formulations are preferably arranged so that each metered dose or "puff" of aerosol contains 20µg-2000µg, preferably about 20µg-500µg of a compound of formula (I). Administration may be once daily or several times daily, for example 2, 3, 4 or 8 times, giving for example 1, 2 or 3 doses each time. The overall daily dose with an aerosol will be within the range 100µg-10mg preferably, 200µg-2000µg. The overall daily dose and the metered dose delivered by capsules and cartridges in an inhaler or insufflator will generally be double those with aerosol formulations.

Topical preparations may be administered by one or more applications per day to the affected area; over skin areas occlusive dressings may advantageously be used. Continuous or prolonged delivery may be achieved by an adhesive reservoir system.

For internal administration the compounds according to the invention may, for example, be formulated in conventional manner for oral, parenteral or rectal administration. Formulations for oral administration include syrups, elixirs, powders, granules, tablets and capsules which typically contain conventional excipients such as binding agents, fillers, lubricants, disintegrants, wetting agents, suspending agents, emulsifying agents, preservatives, buffer salts, flavouring, colouring and/or sweetening agents as appropriate. Dosage unit forms are, however, preferred as described below.

Preferred forms of preparation for internal administration are dosage unit forms i.e. tablets and capsules. Such dosage unit forms contain from 0.1mg to 20mg preferably from 2.5 to 10mg of the compounds of the invention.

The compounds according to the invention may in general be given by internal administration in cases where systemic adreno-cortical therapy is indicated.

In general terms preparations, for internal administration may contain from 0.05 to 10% of the active ingredient dependent upon the type of preparation involved. The daily dose may vary from 0.1 mg to 60mg, e.g. 5-30mg, dependent on the condition being treated, and the duration of treatment desired.

Slow release or enteric coated formulations may be advantageous, particularly for the treatment of inflammatory bowel disorders.

The compound and pharmaceutical formulations according to the invention may be used in combination with or include one or more other therapeutic agents, for example selected from anti-inflammatory agents, anticholinergic agents (particularly an M₁/M₂/M₃ receptor antagonist), β₂₋adrenoreceptor agonists, antiinfective agents (e.g. antibiotics, antivirals), or antihistamines. The invention thus provides, in a further aspect, a combination comprising a compound of formula (I) or a pharmaceutically acceptable salt, solvate or physiologically functional derivative thereof together with one or more other therapeutically active agents, for example selected from an anti-inflammatory agent (for example another corticosteroid or an NSAID), an anticholinergic agent, a β₂₋adrenoreceptor agonist, an antiinfective agent (e.g. an antibiotic or an antiviral), or an antihistamine. Combinations comprising a compound of formula (I) or a pharmaceutically acceptable salt, solvate or physiologically functional derivative thereof together with a β₂-adrenoreceptor agonist, and/or an anticholinergic, and/or a PDE-4 inhibitor are preferred. Preferred combinations are those comprising one or two other therapeutic agents.

It will be clear to a person skilled in the art that, where appropriate, the other therapeutic ingredient(s) may be used in the form of salts, (e.g. as alkali metal or amine salts or as acid addition salts), or prodrugs, or as esters (e.g. lower alkyl esters), or as solvates (e.g. hydrates) to optimise the activity and/or stability and/or physical characteristics (e.g. solubility) of the therapeutic ingredient. It will be clear also that where appropriate, the therapeutic ingredients may be used in optically pure form.

A combination comprising of compound of the invention together with a β₂-adrenoreceptor agonist is particularly preferred.

Examples of β₂-adrenoreceptor agonists include salmeterol (e.g. as racemate or a single enantiomer such as the R-enantiomer), salbutamol, formoterol, salmefamol, fenoterol or terbutaline and salts thereof, for example the xinafoate salt of salmeterol, the sulphate salt or free base of salbutamol or the fumarate salt of formoterol. Long-acting β₂-adrenoreceptor agonists are preferred, especially those having a therapeutic effect over a 24 hour period.

Further examples of β₂-adrenoreceptor agonists include carmoterol, etanterol, naminterol, clenbuterol, pirbuterol, flerobuterol, reproterol, bambuterol, indacaterol and salts thereof.

Preferred β₂-adrenoreceptor agonists include those described in WO 02/066422, WO 02/070490, WO 02/076933, WO 03/024439, WO 03/072539, WO 03/091204, WO 04/016578, WO 2004/022547, WO 2004/037807, WO 2004/037773, WO 2004/037768, WO 2004/039762, WO 2004/039766, WO01/42193 and WO003/042160.

Especially preferred β₂-adrenoreceptor agonists include compounds of formula (XX): 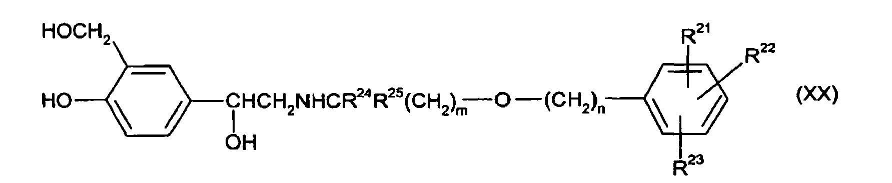 or a salt or solvate thereof, wherein:
m is an integer of from 2 to 8;
n is an integer of from 3 to 11,
with the proviso that m + n is 5 to 19,
R²¹ is -XSO₂NR²⁶R²⁷ wherein X is -(CH₂)p- or C₂₋₆ alkenylene;
R²⁶ and R²⁷ are independently selected from hydrogen, C₁₋₆alkyl, C₃₋₇cycloalkyl C(O)NR²⁸R²⁹, phenyl, and phenyl (C₁₋₄alkyl)-,
or R²⁶ and R²⁷, together with the nitrogen to which they are bonded, form a 5-, 6-, or 7- membered nitrogen containing ring, and R²⁶ and R²⁷ are each optionally substituted by one or two groups selected from halo, C₁-₆alkyl, C₁-₆haloalkyl, C₁₋₆alkoxy, hydroxy-substituted C₁₋₆alkoxy, -CO₂R²⁸ -SO₂NR²⁸R²⁹, -CONR²⁸R²⁹,
   -NR²⁸C(O)R²⁹, or a 5-, 6- or 7-membered heterocylic ring;
R²⁸ and R²⁹ are independently selected from hydrogen, C₁₋₆alkyl, C₃₋₆cycloalkyl , phenyl, and phenyl (C₁₋₄alkyl)-; and
p is an integer of from 0 to 6, preferably from 0 to 4;
R²² and R²³ are independently selected from hydrogen, C₁₋₆alkyl, C₁₋₆alkoxy, halo, phenyl, and C₁₋₆haloalkyl; and
R²⁴ and R²⁵ are independently selected from hydrogen and C₁₋₄alkyl with the proviso that the total number of carbon atoms in R²⁴ and R²⁵ is not more than 4.

Especially preferred β₂-adrenoreceptor agonists are:
3-(4-{[6-({(2R)2-hydroxy-2-[4-hydroxy-3-(hydroxymethyl)phenyl]ethyl}amino) hexyl]oxy}butyl)benzenesulfonamide;
3-(3-{[7-({(2R)-2-hydroxy-2-[4-hydroxy-3-hydroxymethyl)phenyl]ethyl}-amino)heptyl]oxy}propyl)benzenesulfonamide;
4-{(1 R)-2-[(6-{2-[(2,6-dichlorobenzyl)oxy]ethoxy)hexyl)amino]-1-hydroxyethyl)-2-(hydroxymethyl)phenol;
4-{(1*R*)-2-[(6-{4-[3-(cyclopentylsulfonyl)phenyl]butoxy}hexyl)amino]-1-hydroxyethyl}-2-(hydroxymethyl)phenol;
N-[2-hydroxyl-5-[(1R)-1-hydroxy-2-[[2-4-[[(2R)-2-hydroxy-2-phenylethyl]amino]phenyl]ethyl]amino]ethyl]phenyl]foramide;
N-2{2-[4-(3-phenyl-4-methoxyphenyl)aminophenyl]ethyl}-2-hydroxy-2-(8-hydroxy-2(1*H*)-quinolinon-5-yl)ethylamine; and
5-{(*R*)-2-(2-{4-[4-(2-amino-2-methyl-propoxy)-phenylamino]phenyl}-ethylamino)-1-hydroxy-ethyl}-8-hydroxy-1 H-quinolin-2-one.

Suitable anti-inflammatory agents include corticosteroids. Suitable corticosteroids which may be used in combination with the compounds of the invention are those oral and inhaled corticosteroids and their pro-drugs which have anti-inflammatory activity. Examples include methyl prednisolone, prednisolone, dexamethasone, fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid *S-*fluoromethyl ester, 6α,9α-difluoro-17α-[(2-furanylcarbonyl)oxy]-11β-hydroxy-16α-methyl-3-oxo-androsta-1,4-diene-17β-carbothioic acid S-fluoromethyl ester, 6α,9α-difluoro-11β-hydroxy-16α-methyl-3-oxo-17α-propionyloxy- androsta-1,4-diene-17β-carbothioic acid S-(2-oxo-tetrahydro-furan-3S-yl) ester, beclomethasone esters (eg. the 17-propionate ester or the 17,21-dipropionate ester), budesonide, flunisolide, mometasone esters (eg. the furoate ester), triamcinolone acetonide, rofleponide, ciclesonide (16α,17-[[(R)-cyclohexylmethylene]bis(oxy)]-11β,21-dihydroxy-pregna-1,4-diene-3,20-dione), butixocort propionate, RPR-106541, and ST-126. Preferred corticosteroids include fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester and 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester, more preferably 6α,9α-difluoro-17α-[(2-furanylcarbonyl)oxy]-11β-hydroxy-16α-methyl-3-oxo-androsta-1,4-diene-17α-carbothioic acid S-fluoromethyl ester. Further examples of corticosteroids include 6α,9α-difluoro-11β-hydroxy-16α-methyl-3-oxo-17α-(2,2,3,3-tetramethycyclopropylcarbonyl)oxy-androsta-1,4-diene-17β-carbothioic acid S-cyanomethyl ester and 6α,9α-difluoro-11β-hydroxy-16α-methyl-17α-(1-methycyclopropylcarbonyl)oxy-3-oxo-androsta-1,4-diene-17β-carbothioic acid S-fluoromethyl ester.

Non-steroidal compounds having glucocorticoid agonism that may possess selectivity for transrepression over transactivation and that may be useful in combination therapy include those covered in the following patents: WO03/082827, WO01/10143 WO98/54159, WO04/005229 WO04/009016 WO04/009017 WO04/018429, WO03/104195, WO03/082787, WO03/082280, WO03/059899, WO03/101932, WO02/02565, WO01/16128, WO00/66590, WO03/086294 WO04/026248, WO03/061651 WO03/08277.

Suitable anti-inflammatory agents include non-steroidal anti-inflammatory drugs (NSAID's).

Suitable NSAID's include sodium cromoglycate, nedocromil sodium, phosphodiesterase (PDE) inhibitors (e.g. theophylline, PDE4 inhibitors or mixed PDE3/PDE4 inhibitors), leukotriene antagonists, inhibitors of leukotriene synthesis (eg. montelukast), iNOS inhibitors, tryptase and elastase inhibitors, beta-2 integrin antagonists and adenosine receptor agonists or antagonists (e.g. adenosine 2a agonists), cytokine antagonists (e.g. chemokine antagonists, such as a CCR3 antagonist) or inhibitors of cytokine synthesis, or 5-lipoxygenase inhibitors. Suitable other β₂₋adrenoreceptor agonists include salmeterol (e.g. as the xinafoate), salbutamol (e.g. as the sulphate or the free base), formoterol (e.g. as the fumarate), fenoterol or terbutaline and salts thereof. An iNOS (inducible nitric oxide synthase inhibitor) is preferably for oral administration. Suitable iNOS inhibitors include those disclosed in WO93/13055, WO98/30537, WO02/50021, WO95/34534 and WO99/62875. Suitable CCR3 inhibitors include those disclosed in WO02/26722.

Of particular interest is use of the compounds of formula (I) in combination with a phosphodiesterase 4 (PDE4) inhibitor, especially in the case of a formulation adapted for inhalation. The PDE4-specific inhibitor useful in this aspect of the invention may be any compound that is known to inhibit the PDE4 enzyme or which is discovered to act as a PDE4 inhibitor, and which are only PDE4 inhibitors, not compounds which inhibit other members of the PDE family, such as PDE3 and PDE5, as well as PDE4.

Compounds of interest include cis-4-cyano-4-(3-cyclopentyloxy-4-methoxyphenyl)cyclohexan-1-carboxylic acid, 2-carbomethoxy-4-cyano-4-(3-cyclopropylmethoxy-4-difluoromethoxyphenyl)cyclohexan-1-one and *cis*-[4-cyano-4-(3-cyclopropylmethoxy-4-difluoromethoxyphenyl)cyclohexan-1-ol]. Also, *cis*-4-cyano-4-[3-(cyclopentyloxy)-4-methoxyphenyl]cyclohexane-1-carboxylic acid (also known as cilomilast) and its salts, esters, pro-drugs or physical forms, which is described in U.S. patent 5,552,438 issued 03 September, 1996.

Other compounds of interest include AWD-12-281 from Elbion (Hofgen, N. et al. 15th EFMC lnt Symp Med Chem (Sept 6-10, Edinburgh) 1998, Abst P.98; CAS reference No. 247584020-9); a 9-benzyladenine derivative nominated NCS-613 (INSERM); D-4418 from Chiroscience and Schering-Plough; a benzodiazepine PDE4 inhibitor identified as Cl-1018 (PD-168787) and attributed to Pfizer; a benzodioxole derivative disclosed by Kyowa Hakko in WO99/96766; K-34 from Kyowa Hakko; V-11294A from Napp (Landells, L.J. et al. Eur Resp J [Annu Cong Eur Resp Soc (Sept 19-23, Geneva) 1998] 1998, 12 (Suppl. 28): Abst P2393); roflumilast (CAS reference No 162401-32-3) and a pthalazinone (WO99/47505) from Byk-Gulden; Pumafentrine, (-)-p-[(4aR^{*},10*b*S^{*})-9-ethoxy-1,2,3,4,4a,10b-hexahydro-8-methoxy-2-methylbenzo[c][1,6]naphthyridin-6-yl]-N,N-diisopropylbenzamide which is a mixed PDE3/PDE4 inhibitor which has been prepared and published on by Byk-Gulden, now Altana; arofylline under development by Almirall-Prodesfarma; VM554/UM565 from Vernalis; or T-440 (Tanabe Seiyaku; Fuji, K. et al. J Pharmacol Exp Ther, 1998, 284(1): 162), and T2585.

Further compounds of interest are disclosed in the published international patent application WO04/024728 (Glaxo Group Ltd), PCT/EP2003/014867 (Glaxo Group Ltd) and PCT/EP2004/005494 (Glaxo Group Ltd).

Suitable anticholinergic agents are those compounds that act as antagonists at the muscarinic receptors, in particular those compounds which are antagonists of the M₁ or M₃ receptors, dual antagonists of the M₁/M₃ or M₂/M₃, receptors or pan-antagonists of the M₁/M₂/M₃ receptors. Exemplary compounds for administration via inhalation include ipratropium (e.g. as the bromide, CAS 22254-24-6, sold under the name Atrovent), oxitropium (e.g. as the bromide, CAS 30286-75-0) and tiotropium (e.g. as the bromide, CAS 136310-93-5, sold under the name Spiriva). Also of interest are revatropate (e.g. as the hydrobromide, CAS 262586-79-8) and LAS-34273 which is disclosed in WO01/04118. Exemplary compounds for oral administration include pirenzepine (CAS 28797-61-7), darifenacin (CAS 133099-04-4, or CAS 133099-07-7 for the hydrobromide sold under the name Enablex), oxybutynin (CAS 5633-20-5, sold under the name Ditropan), terodiline (CAS 15793-40-5), tolterodine (CAS 124937-51-5, or CAS 124937-52-6 for the tartrate, sold under the name Detrol), otilonium (e.g. as the bromide, CAS 26095-59-0, sold under the name Spasmomen), trospium chloride (CAS 10405-02-4) and solifenacin (CAS 242478-37-1, or CAS 242478-38-2 for the succinate also known as YM-905 and sold under the name Vesicare).

Other suitable anticholinergic agents include compounds of formula (XXI), which are disclosed in US patent application 60/487981: 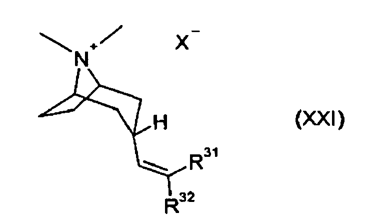 in which the preferred orientation of the alkyl chain attached to the tropane ring is endo;
R³¹ and R³² are, independently, selected from the group consisting of straight or branched chain lower alkyl groups having preferably from 1 to 6 carbon atoms, cycloalkyl groups having from 5 to 6 carbon atoms, cycloalkyl-alkyl having 6 to 10 carbon atoms, 2-thienyl, 2-pyridyl, phenyl, phenyl substituted with an alkyl group having not in excess of 4 carbon atoms and phenyl substituted with an alkoxy group having not in excess of 4 carbon atoms;
**X⁻** represents an anion associated with the positive charge of the N atom. X⁻ may be but is not limited to chloride, bromide, iodide, sulfate, benzene sulfonate, and toluene sulfonate,
   including, for example:
   (3-*endo*)-3-(2,2-di-2-thienylethenyl)-8,8-dimethyl-8-azoniabicyclo[3.2.1]octane bromide;
   (3-*endo*)-3-(2,2-diphenylethenyl)-8,8-dimethyl-8-azoniabicyclo[3.2.1]octane bromide;
   (3-*endo*)-3-(2,2-diphenylethenyl)-8,8-dimethyl-8-azoniabicyclo[3.2.1]octane 4-methylbenzenesulfonate;
   (3-*endo*)-8,8-dimethyl-3-[2-phenyl-2-(2-thienyl)ethenyl]-8-azoniabicyclo[3.2.1]octane bromide; and/or
   (3-endo)-8,8-dimethyl-3-[2-phenyl-2-(2-pyridinyl)ethenyl]-8-azoniabicyclo[3.2.1]octane bromide.

Further suitable anticholinergic agents include compounds of formula (XXII) or (XXIII), which are disclosed in US patent application 60/511009: 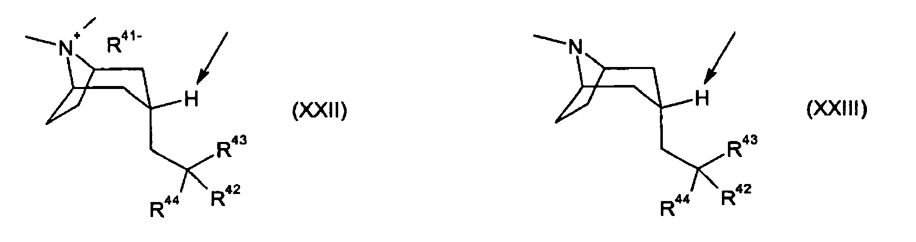 wherein:
the H atom indicated is in the exo position;
R⁴¹ represents an anion associated with the positive charge of the N atom. R⁴¹ may be but is not limited to chloride, bromide, iodide, sulfate, benzene sulfonate and toluene sulfonate;
R⁴² and R⁴³ are independently selected from the group consisting of straight or branched chain lower alkyl groups (having preferably from 1 to 6 carbon atoms), cycloalkyl groups (having from 5 to 6 carbon atoms), cycloalkyl-alkyl (having 6 to 10 carbon atoms), heterocycloalkyl (having 5 to 6 carbon atoms) and N or O as the heteroatom, heterocycloalkyl-alkyl (having 6 to10 carbon atoms) and N or O as the heteroatom, aryl, optionally substituted aryl, heteroaryl, and optionally substituted heteroaryl;
R⁴⁴ is slected from the group consisting of (C₁-C₆)alkyl, (C₃-C₁₂)cycloalkyl, (C₃-C₇)heterocycloalkyl, (C₁-C₆)alkyl(C₃-C₁₂)cycloalkyl, (C₁-C₆)alkyl(C₃-C₇)heterocycloalkyl, aryl, heteroaryl, (C₁-C₆)alkyl-aryl, (C₁-C₆)alkyl-heteroaryl, -OR⁴⁵, -CH₂OR⁴⁵, -CH₂OH, -CN, -CF₃, -CH₂O(CO)R⁴⁶, -CO₂R⁴⁷, -CH₂NH₂, - CH₂N(R⁴⁷)SO₂R⁴⁵, -SO₂N(R⁴⁷)(R⁴⁸), -CON(R⁴⁷)(R⁴⁸), -CH₂N(R⁴⁸)CO(R⁴⁶), - CH₂N(R⁴⁸)SO₂(R⁴⁶), -CH₂N(R⁴⁸)CO₂(R⁴⁵), -CH₂N(R⁴⁸)CONH(R⁴⁷);
R⁴⁵ is selected from the group consisting of (C₁-C₆)alkyl, (C₁-C₆)alkyl(C₃-C₁₂)cycloalkyl, (C₁-C₆)alkyl(C₃-C₇)heterocycloalkyl, (C₁-C₆)alkyl-aryl, (C₁-C₆)alkyl-heteroaryl;
R⁴⁶ is selected from the group consisting of (C₁-C₆)alkyl, (C₃-C₁₂)cycloalkyl, (C₃-C₇)heterocycloalkyl, (C₁-C₆)alkyl(C₃-C₁₂)cycloalkyl, (C₁-C₆)alkyl(C₃-C₇)heterocycloalkyl, aryl, heteroaryl, (C₁-C₆)alkyl-aryl, (C₁-C₆)alkyl-heteroaryl;
R⁴⁷ and R⁴⁸ are, independently, selected from the group consisting of H, (C₁-C₈)alkyl, (C₃-C₁₂)cycloalkyl, (C₃-C₇)heterocycloalkyl, (C₁-C₆)alkyl(C₃-C₁₂)cycloalkyl, (C₁-C₆)alkyl(C₃-C₇)heterocycloalkyl, (C₁-C₆)alkyl-aryl, and (C₁-C₆)alkyl-heteroaryl, including, for example:
   (Endo)-3-(2-methoxy-2,2-di-thiophen-2-yl-ethyl)8,8-dimethyl-8-azonia-bicyclo[3.2.1]octane iodide;
   3-((Endo)-8-methyl-8-aza-bicyclo[3.2.1]oct-3-yl)-2,2-diphenyl-propionitrile;
   (Endo)-8-methyl-3-(2,2,2-triphenyl-ethyl)-8-aza-bicyclo[3.2.1]octane;
   3-((Endo)-8-methyl-8-aza-bicyclo[3.2.1]oct-3-yl)-2,2-diphenyl-propionamide;
   3-((Endo)-8-methyl-8-aza-bicyclo[3.2.1]oct-3-yl)-2,2-diphenyl-propionic acid;
   (Endo)-3-(2-cyano-2,2-diphenyl-ethyl)-8,8-dimethyl-8-azonia-bicyclo[3.2.1]octane iodide;
   (Endo)-3-(2-cyano-2,2-diphenyl-ethyl)-8,8-dimethyl-8-azonia-bicyclo[3.2.1]octane bromide;
   3-((Endo)-8-methyl-8-aza-bicyclo[3.2.1]oct-3-yl)-2,2-diphenyl-propan-1-ol;
   N-Benzyl-3-((endo)-8-methyl-8-aza-bicyclo[3.2.1]oct-3-yl)-2,2-diphenyl-propionamide;
   (Endo)-3-(2-carbamoyl-2,2-diphenyl-ethyl)-8,8-dimethyl-8-azonia-bicyclo[3.2.1]octane iodide;
   1-Benzyl-3-[3-((endo)-8-methyl-8-aza-bicyclo[3.2.1]oct-3-yl)-2,2-diphenyl-propyl]-urea;
   1-Ethyl-3-[3-((endo)8-methyl-8-aza-bicyclo[3.2.1]oct-3-yl)-2,2-diphenyl-propyl]-urea;
   *N*-[3-((Endo)-8-methyl-8-aza-bicyclo[3.2.1]oct-3-yl)-2,2-diphenyl-propyl]-acetamide;
   *N*-[3-((Endo)-8-methyl-8-aza-bicyclo[3.2.1]oct-3-yl)-2,2-diphenyl-propyl]-benzamide;
   3-((Endo)-8-methyl-8-aza-bicyclo[3.2.1]oct-3-yl)-2,2-di-thiophen-2-yl-propionitrile;
   (Endo)-3-(2-cyano-2,2-di-thiophen-2-yl-ethyl)-8,8-dimethyl-8-azonia-bicyclo[3.2.1]octane iodide;
   N-[3-((Endo)-8-methyl-8-aza-bicyclo[3.2.1]oct-3-yl)-2,2-diphenyl-propyl]-benzenesulfonamide;
   [3-((Endo)-8-methyl-8-aza-bicyclo[3.2.1]oct-3-yl)-2,2-diphenyl-propyl]-urea;
   N-[3-((Endo)-8-methyl-8-aza-bicyclo[3.2.1]oct-3-yl)-2,2-diphenyl-propyl]-methanesulfonamide; and/or
   (Endo)-3-{2,2-diphenyl-3-[(1-phenyl-methanoyl)-amino]-propyl}-8,8-dimethyl-8-azonia-bicyclo[3.2.1]octane bromide.

More preferred compounds useful in the present invention include:
(Endo)-3-(2-methoxy-2,2-di-thiophen-2-yl-ethyl)-8,8-dimethyl-8-azonia-bicyclo[3.2.1]octane iodide;
(Endo)-3-(2-cyano-2,2-diphenyl-ethyl)-8,8-dimethyl-8-azonia-bicyclo[3.2.1]octane iodide;
(Endo)-3-(2-cyano-2,2-diphenyl-ethyl)-8,8-diimethyl-8-azonia-bicyclo[3.2.1]octane bromide;
(Endo)-3-(2-carbamoyl-2,2-diphenyl-ethyl)8,8-dimethyl-8-azonia-bicyclo[3.2.1]octane iodide;
(Endo)-3-(2-cyano-2,2-di-thiophen-2-yl-ethyl)-8,8-dimethyl-8-azonia-bicyclo[3.2.1]octane iodide; and/or
(Endo)-3-{2,2-diphenyl-3-[(1-phenyl,methanoyl)-amino]-propyl}-8,8-dimethyl-8-azonia-bicyclo[3.2.1]octane bromide.

Suitable antihistamines (also referred to as H1-receptor antagonists) include any one or more of the numerous antagonists known which inhibit H1-receptors, and are safe for human use. First generation antagonists, include derivatives of ethanolamines, ethylenediamines, and alkylamines, e.g diphenylhydramine, pyrilamine, clemastine, chloropheniramine. Second generation antagonists, which are non-sedating, include loratidine, desloratidine,terfenadine,astemizole,acrivastine, azelastine, levocetirizine fexofenadine and cetirizine.

Examples of preferred anti-histamines include loratidine, desloratidine, fexofenadine and cetirizine.

The invention thus provides, in a further aspect, a combination comprising a compound of formula (I) a pharmaceutically acceptable salt, solvate or physiologically functional derivative thereof together with a PDE4 inhibitor.

The invention thus provides, in a further aspect, a combination comprising a compound of formula (I) a pharmaceutically acceptable salt, solvate or physiologically functional derivative thereof together with a β₂-adrenorecptor agonist.

The invention thus provides, in a further aspect, a combination comprising a compound of formula (I) a pharmaceutically acceptable salt, solvate or physiologically functional derivative thereof together with an anticholinergic.

The invention thus provides, in a further aspect, a combination comprising a compound of formula (I) a pharmaceutically acceptable salt, solvate or physiologically functional derivative thereof together with an antihistamine.

The invention thus provides, in a further aspect, a combination comprising a compound of formula (I) a pharmaceutically acceptable salt, solvate or physiologically functional derivative thereof together with a PDE4 inhibitor and a β₂-adrenoreceptor agonist.

The invention thus provides, in a further aspect, a combination comprising a compound of formula (I) a pharmaceutically acceptable salt, solvate or physiologically functional derivative thereof together with an anticholinergic and a PDE-4 inhibitor.

The combinations referred to above may conveniently be presented for use in the form of a pharmaceutical formulation and thus pharmaceutical formulations comprising a combination as defined above together with a pharmaceutically acceptable diluent or carrier represent a further aspect of the invention.

The individual compounds of such combinations may be administered either sequentially or simultaneously in separate or combined pharmaceutical formulations. Preferably the individual compounds of such combinations may be administered simultaneously in a combined pharmaceutical combination. Appropriate doses of known therapeutic agents will be readily appreciated by those skilled in the art.

The compounds of formula (I) and solvates thereof may be prepared by the methodology described hereinafter, constituting a further aspect of this invention.

A process according to the invention for preparing a compound of formula (I) comprises reaction of a carboxylic acid of formula (II) 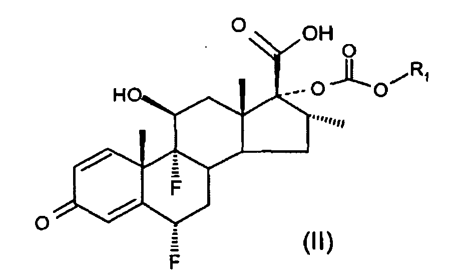 wherein R₁ is as defined above,
with a compound of formula L-CH₂-CN wherein L represents a leaving group.

In this process the compound of formula (II) may be reacted with a compound of formula L-CH₂-CN wherein L represents a leaving group such as halogen atom or a tosyl or mesyl group or the like, under standard conditions. For example the reaction may be performed in an inert polar organic solvent e.g. N,N-dimethylformamide in the presence of a base e.g. potassium carbonate, sodium carbonate.

Compounds of formula (II) may conveniently be employed as salts when such salts may be prepared in crystalline form, or as solvates.

Compounds of formula L-CH₂-CN are either known or may be prepared by known methods.

In a further aspect of the present invention, there is provided a compound of formula (II) 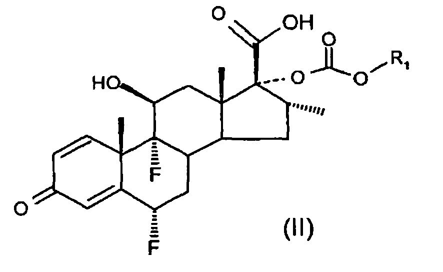 wherein R₁, is as defined for compounds of formula (I)**.**

Compounds of formula (II) may be prepared from the corresponding 17α-hydroxyl derivative of formula (III): 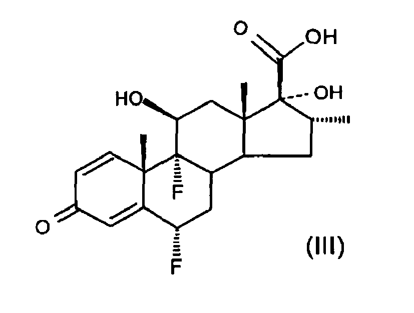 by reaction with a chloroformate R₁OCOCl or preferably an anhydride (R₁OCO)₂O in pyridine solution.

The compound of formula (III) and a process for it's synthesis are described in G. H. Phillipps et al., Journal of Medicinal Chemistry, (1994), 37, 3717-3729.

The following compounds of formula (II) are new and form an aspect of the invention:
(6α,11β,16α,17α)-17-({[(1,1-dimethylethyl)oxy]carbonyl}oxy)-6,9-difluoro-11-hydroxy-16-methyl-3-oxoandrosta-1,4-diene-17-carboxylate;
(6α,11β,16α,17α)-17-({[(1,1-dimethylpropyl)oxy]carbonyl}oxy)-6,9-difluoro-11-hydroxy-16-methyl-3-oxoandrosta-1,4-diene-17-carboxylate.

Compounds of formula (I) and/or solvates thereof demonstrate agonism at the glucocorticoid receptor.

Compounds of formula (I) and/or solvates thereof may demonstrate good anti-inflammatory properties, with predictable pharmacokinetic and pharmacodynamic behaviour. They also may have an attractive side-effect profile, demonstrated, for example, by increased selectivity for the glucocorticoid receptor over the progesterone receptor and/or increased selectivity for glucocorticoid receptor mediated transrepression over transactivation and are likely to be compatible with a convenient regime of treatment in human patients.

The following non-limiting Examples illustrate the invention:

### EXAMPLES

### General

Chromatographic purification was performed using pre-packed Bond Elut silica gel cartridges available commercially from Varian. These cartridges were preconditioned with dichloromethane prior to use. LCMS was conducted on a Supelcosil LCABZ+PLUS column (3.3 cm x 4.6 mm ID) eluting with 0.1% HCO₂H and 0.01 M ammonium acetate in water (solvent A), and 0.05% HCO₂H 5% water in acetonitrile (solvent B), using the following elution gradient 0-0.7 min 0%B, 0.7-4.2 min 100%B, 4.2-5.3 min 0%B, 5.3-5.5 min 0%B at a flow rate of 3 ml/min. The mass spectra were recorded on a Fisons VG Platform spectrometer using electrospray positive and negative mode (ES+ve and ES-ve).

### Intermediates

### Intermediate 1: (6α,11β,16α,17α)-17-({[(1,1-Dimethylethyl)oxy]carbonyl}oxy)-6,9-difluoro-11-hydroxy-16-methyl-3-oxoandrosta-1,4-diene-17-carboxylic acid

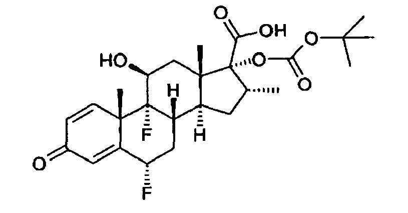

Bis(1,1-dimethylethyl) carbonate (121mg, 0.56mmol) was added to a stirred solution of (6α,11β,16α,17α)-6,9-difluoro-11,17-dihydroxy-16-methyl-3-oxoandrosta-1,4-diene-17-carboxylic acid (G. H. Phillipps et al., (1994) Journal of Medicinal Chemistry, 37, 3717-3729) (200mg, 0.5mmol) in pyridine (5ml) and the mixture stirred at room temperature overnight. The solvent was evaporated and the residue was stirred with 2M hydrochloric acid (20ml) and the resulting precipitate was collected by filtration, washed with water and dried *in* vacuo at 60°C to give the title compound : LCMS retention time 3.27 min.

### Intermediate 2: (6α,11β,6α,17α)-17-({[(1,1-Dimethylpropyl)oxy]carbonyl}oxy)-6,9-difluoro-11-hydroxy-16-methyl-3-oxoandrosta-1,4-diene-17-carboxylic acid

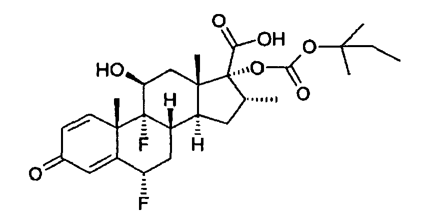

Prepared using method similar to that described for (6α,11β,16α,17α)-17-({[(1,1-Dimethylethyl)oxy]carbonyl}oxy)-6,9-difluoro-11-hydroxy-16-methyl-3-oxoandrosta-1,4-diene-17-carboxylic acid (Intermediate 1). LCMS retention time 3.38 min.

### Examples

### Example 1: Cyanomethyl (6α,11β,16α,17α)-17-({[1.1-dimethylethyl)oxy]carbonyl}oxy)-6,9-difluoro-11-hydroxy-16-methyl-3-oxoandrosta-1,4-diene-17-carboxylate

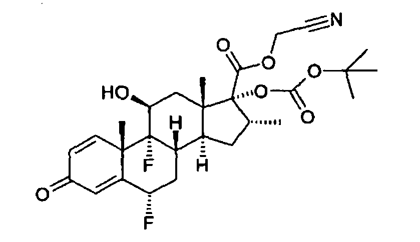

Sodium carbonate (321mg, 3mmol) was added to a solution of (6α,11β,16α,17α)-17-({[(1,1-Dimethylethyl)oxy]carbonyl}oxy)-6,9-difluoro-11-hydroxy-16-methyl-3-oxoandrosta-1,4-diene-17-carboxylic acid (Intermediate 1) (150mg, 0.3mmol) in N,N-dimethylformamide (3ml) under nitrogen and the mixture stirred at room temperature for 15min and then cooled in ice. Bromoacetonitrile (55µl, 0.815mmol) was added and the mixture allowed to warm to room temperature and stirred overnight. Diethylamine (40µl, 0.51 mmol) was added and the mixture was then added dropwise to 2M hydrochloric acid (20ml). The product was extracted into ethyl acetate and the extract was dried and evaporated and purified on a Bond Elut cartridge using a 0-100% cyclohexane/ether gradient to give the title compound (123mg): LCMS retention time 3.51 min, *m*/*z* 536 MH⁺

### Example 2: Cyanomethyl (6α,11β,16α,17α)-17-({[(1,1-dimethylpropyl)oxy]carbonyl}oxy)-6,9-difluoro-11-hydroxy-16-methyl-3-oxoandrosta-1,4-diene-17-carboxylate

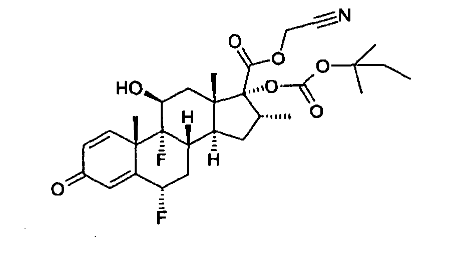

Example 2 was prepared from (6α,11β,16α,17α)-17-({[(1,1-Dimethylpropyl)oxy]carbonyl}oxy)-6,9-difluoro-11-hydroxy-16-methyl-3-oxoandrosta-1,4-diene-17-carboxylic acid (Intermediate 2) using a method similar to that described for Example 1. LCMS retention time 3.59 min, *m*/*z* 550 MH⁺

### Pharmacological Activity

Pharmacological activity may be assessed in functional *in vitro* assays of glucocorticoid agonist activity.

### Assay for Transrepression Activity of the Glucocorticoid Agonists

The functional assay based on that described by K.P.Ray et al., Biochem J. (1997), 328, 707-715 provides a measure of transrepressive activity of a glucocorticoid agonist. A549 cells stably transfected with a reporter gene containing the NF-κB responsive elements from the ELAM gene promoter coupled to sPAP (secreted alkaline phosphatase) are treated with test compounds at appropriate doses for 1 hour at 37°C. The cells are then stimulated with tumour necrosis factor (TNF, 10ng/ml) for 16 hours, at which time the amount of alkaline phosphatase produced is measured by a standard colourimetric assay. Dose response curves were constructed from which EC₅₀ values were estimated.

The pEC₅₀ values of Examples 1 and 2 were >10 in this assay.

### Assay for Transactivation Activity of the Glucocorticoid Agonists.

The functional assay based on that described by R.J.H. Austin et al., Eur Resp J. (2002), 20,1386-1392 measures the ability of compounds to directly transactivate gene expression. A549 cells stably transfected with a reporter gene containing the glucocorticoid responsive region of the mouse mammary tumour virus long terminal repeat (MMTV-LTR) coupled to renilla luciferase were treated with test compounds at appropriate doses for 6 hour at 37°C. The amount of luciferase activity present within the cells is then determined by measuring the light emitted following incubation with a suitable substrate. Dose response curves were constructed from which EC₅₀ values were estimated and from which maximal responses are calculated relative to Dexamethasone (100%).

Compounds of Examples 1 and 2 showed maximal responses of <10% in this assay.

### Assay for Progesterone Receptor Activity

A T225 flask of CV-1 cells at a density of 80% confluency was washed with PBS, detached from the flask using 0.25% trypsin and counted using a Sysmex KX-21 N. Cells were diluted in DMEM containing 10% Hyclone, 2mM L-Glutamate and 1% Pen/Strep at 140 cells/µl and transduced with 10% PRb-BacMam and 10% MMTV-BacMam. 70 ml of suspension cells were dispensed to each well of white Nunc 384-well plates, containing compounds at the required concentration. After 24h 10 µl of Steady Glo were added to each well of the plates. Plates were incubated in the dark for 10 min before reading them on a Viewlux reader. Dose response curves were constructed from which pEC₅₀ values were estimated.

The pEC₅₀ values for compounds of Examples 1 and 2 were <7 in this assay.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

## Claims

1. A compound of formula (I): 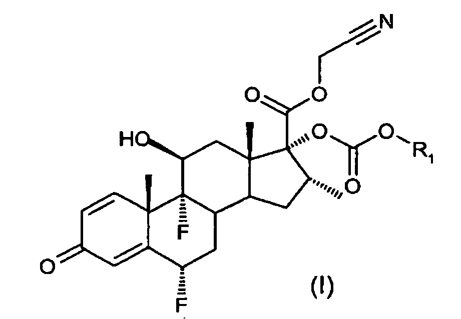 wherein
R₁ represents a 1,1-dimethylethyl group ora 1,1-dimethylpropyl group;
or a physiologically acceptable solvate thereof.

2. A compound as claimed in claim 1 which is cyanomethyl (6α,11β,16α,17α)-17-({[(1,1-dimethylpropyl)oxy]carbonyl}oxy)-6,9-difluoro-11-hydroxy-16-methyl-3-oxoandrosta-1,4-diene-17-carboxylate.

3. A compound as claimed in claim 1 which is cyanomethyl (6α,11β,16α,17α)-17-({[(1,1-dimethylethyl)oxy]carbonyl}oxy)-6,9-difluoro-11-hydroxy-16-methyl-3-oxoandrosta-1,4-diene-17-carboxylate.

4. A compound of formula (I) as defined in any one of claims 1 to 3 or a physiologically acceptable solvate thereof for use in veterinary or human medicine.

5. Use of a compound of formula (I) as defined in any one of claims 1 to 3 or a physiologically acceptable solvate thereof for the manufacture of a medicament for the treatment of inflammatory and/or allergic conditions.

6. A pharmaceutical composition comprising a compound of formula (I) as defined in any one of claims 1 to 3 or a physiologically acceptable solvate thereof together, if desirable, in admixture with one or more physiologically acceptable diluents or carriers.

7. A pharmaceutical aerosol formulation comprising a compound of formula (I) as defined in any one of claims 1 to 3 or a physiologically acceptable solvate thereof, and a fluorocarbon or hydrogen-containing chlorofluoro carbon as propellant, optionally in combination with a surfactant and/or a cosolvent.

8. A pharmaceutical composition according to claim 7 which further comprises another therapeutically active agent.

9. A pharmaceutical composition according to claim 8 in which said another therapeutically active agent is a β₂-adrenoreceptor agonist.

10. A compound which is
(6α,11β,16α,17α)-17-({[(1,1-dimethylethyl)oxy]carbonyl}oxy)-6,9-difluoro-11-hydroxy-16-methyl-3-oxoandrosta-1,4-diene-17-carboxylic acid.

11. A compound which is
(6α,11β,16α,17α)-17-({[(1,1-dimethylpropyl)oxy]carbonyl}oxy)-6,9-difluoro-11-hydroxy-16-methyl-3-oxoandrosta-1,4-diene-17-carboxylic acid.

12. A process for preparing a compound of formula (I) which comprises reaction of a carboxylic acid of formula (II); 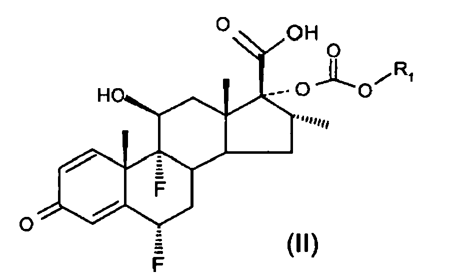 wherein R₁ is as defined in any one of claims 1 to 3;
with a compound of formula L-CH₂-CN wherein L represents a leaving group.

13. A process for preparing a compound of formula (II) 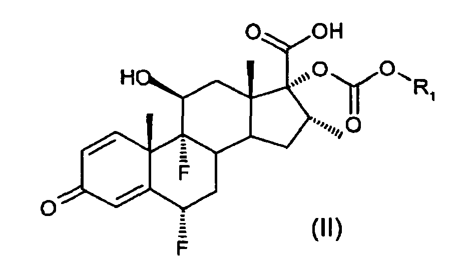 wherein R₁ is as defined in any one of claims 1 to 3 which process comprises reaction of a chloroformate R₁OCOCl or an anhydride (R₁OCO)₂O with the corresponding 17α-hydroxyl derivative of formula (III):

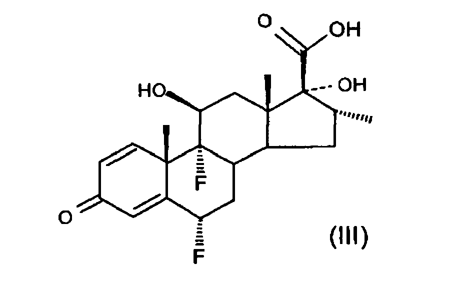

## Patentansprüche

1. Verbindung der Formel (I): 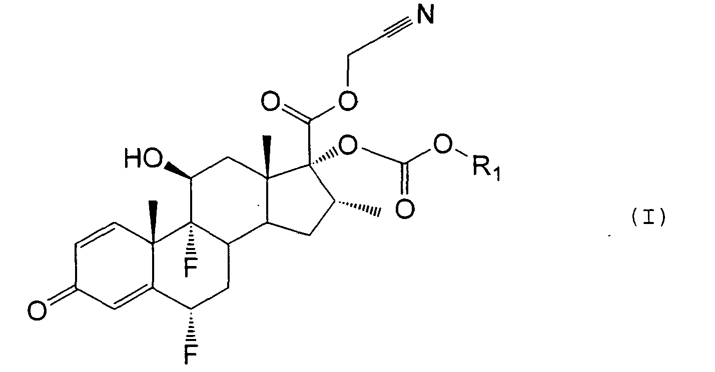 worin
R₁ eine 1,1-Dimethylethylgruppe oder eine 1,1-Dimethylpropylgruppe darstellt;
oder ein physiologisch annehmbares Solvat davon.

2. Verbindung gemäß Anspruch 1, die Cyanomethyl-(6α,11β,16α,17α)-17-({[(1,1-dimethylpropyl)oxy]-carbonyl}oxy)-6,9-difluor-11-hydroxy-16-methyl-3-oxoandrosta-1,4-dien-17-carboxylat ist.

3. Verbindung gemäß Anspruch 1, die Cyanomethyl-(6α,11β,16α,17α)-17-({[(1,1-dimethylethyl)oxy]-carbonyl}oxy)-6,9-difluor-11-hydroxy-16-methyl-3-oxoandrosta-1,4-dien-17-carboxylat ist.

4. Verbindung der Formel (I) gemäß einem der Ansprüche 1 bis 3 oder ein physiologisch annehmbares Solvat davon zur Verwendung in der Veterinär- oder Humanmedizin.

5. Verwendung einer Verbindung der Formel (I) gemäß einem der Ansprüche 1 bis 3 oder eines physiologisch annehmbaren Solvats davon in der Herstellung eines Medikaments zur Behandlung von Entzündungs- und/oder allergischen Zuständen.

6. Pharmazeutische Zusammensetzung, die eine Verbindung der Formel (I) gemäß einem der Ansprüche 1 bis 3 oder ein physiologisch annehmbares Solvat davon zusammen, falls erwünscht, im Gemisch mit einem oder mehreren physiologisch annehmbaren Verdünnungsstoffen oder Trägern umfaßt.

7. Pharmazeutische Aerosolformulierung, die eine Verbindung der Formel (I) gemäß einem der Ansprüche 1 bis 3 oder ein physiologisch annehmbares Solvat davon und einen Fluorkohlenstoff oder Wasserstoff-haltigen Chlorfluorkohlenstoff als Treibmittel, gegebenenfalls in Kombination mit einem Tensid und/oder einem Co-Lösungsmittel umfaßt.

8. Pharmazeutische Zusammensetzung gemäß Anspruch 7, die ferner ein anderes therapeutisch wirksames Mittel umfaßt.

9. Pharmazeutische Zusammensetzung gemäß Anspruch 8, in der das andere therapeutisch wirksame Mittel ein β₂-Adrenorezeptoragonist ist.

10. Verbindung, die (6α,11β,16α,17α)-17-({[(1,1-dimethylethyl)oxy]carbonyl}oxy)-6,9-difluor-11-hydroxy-16-methyl-3-oxoandrosta-1,4-dien-17-carbonsäure ist.

11. Verbindung, die (6α,11β,16α,17α)-17-({[(1,1-dimethylpropyl)oxy]carbonyl}oxy)-6,9-difluor-11-hydroxy-16-methyl-3-oxoandrosta-1,4-dien-17-carbonsäure ist.

12. Verfahren zur Herstellung einer Verbindung der Formel (I), das das Umsetzen einer Carbonsäure der Formel (II): 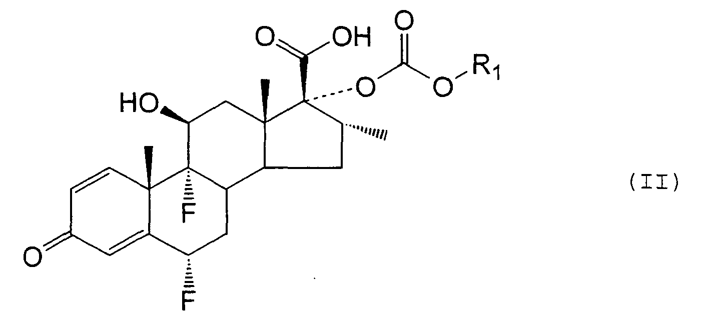 worin R₁ wie in einem der Ansprüche 1 bis 3 definiert ist,
mit einer Verbindung der Formel L-CH₂-CN, worin L eine Abgangsgruppe darstellt, umfaßt.

13. Verfahren zur Herstellung einer Verbindung der Formel (II) : 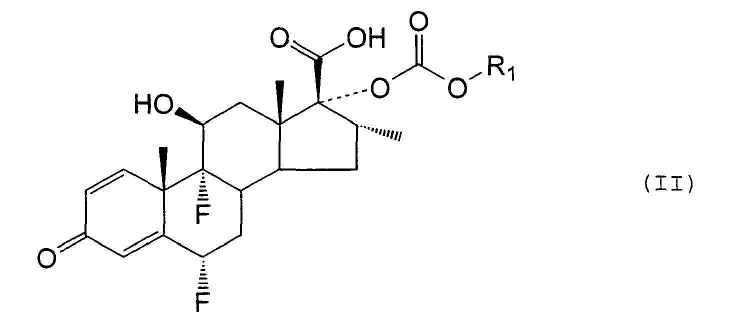 worin R₁ wie in einem der Ansprüche 1 bis 3 definiert ist,
wobei das Verfahren das Umsetzen eines Chlorformiats R₁OCOCl oder eines Anhydrids (R₁OCO)₂O mit dem entsprechenden 17α-Hydroxylderivat der Formel (III): 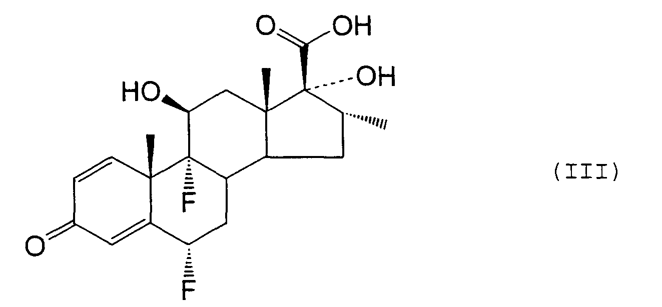 umfaßt.

## Revendications

1. Composé de formule (I) 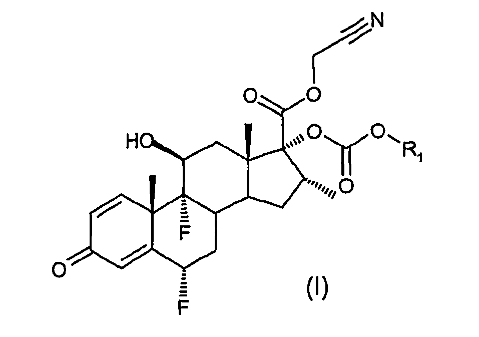 dans laquelle
R₁ représente un groupe 1,1-diméthyléthyle ou un groupe 1,1-diméthylpropyle ; ou solvate physiologiquement acceptable de celui-ci.

2. Composé selon la revendication 1 qui est un (6α,11β,16α,17α)-17-({[(1,1-diméthylpropyl)oxy]carbonyl}oxy)-6,9-difluoro-11-hydroxy-16-méthyl-3-oxoandrosta-1,4-diène-17-carboxylate de cyanométhyle.

3. Composé selon la revendication 1 qui est un (6α,11β,16α,17α)-17-({[(1,1-diméthyléthyl)oxy]carbonyl}oxy)-6,9-difluoro-11-hydroxy-16-méthyl-3-oxoandrosta-1,4-diène-17-carboxylate de cyanométhyle.

4. Composé de formule (I) tel que défini dans l'une quelconque des revendications 1 à 3 ou solvate physiologiquement acceptable de celui-ci utilisable en médecine vétérinaire ou humaine.

5. Utilisation d'un composé de formule (I) tel que défini dans l'une quelconque des revendications 1 à 3 ou d'un solvate physiologiquement acceptable de celui-ci pour la fabrication d'un médicament pour le traitement d'affections inflammatoires et/ou allergiques.

6. Composition pharmaceutique comprenant un composé de formule (I) tel que défini dans l'une quelconque des revendications 1 à 3 ou un solvate physiologiquement acceptable de celui-ci, ensemble si souhaitable, en mélange avec un ou plusieurs diluants ou véhicules physiologiquement acceptables.

7. Formulation pharmaceutique de type aérosol comprenant un composé de formule (I) tel que défini dans l'une quelconque des revendications 1 à 3 ou un solvate physiologiquement acceptable de celui-ci, et un fluorocarbone ou un chlorofluorocarbone contenant de l'hydrogène à titre de propulseur, éventuellement en combinaison avec un tensioactif et/ou un co-solvant.

8. Composition pharmaceutique selon la revendication 7 qui comprend, en outre, un autre agent thérapeutiquement actif.

9. Composition pharmaceutique selon la revendication 8, dans laquelle ledit autre agent thérapeutiquement actif est un agoniste des β₂-adrénorécepteurs.

10. Composé qui est un acide (6α,11β,16α,17α)-17-({[(1,1-diméthyléthyl)oxy]carbonyl}oxy)-6,9-difluoro-11-hydroxy-16-méthyl-3-oxoandrosta-1,4-diène-17-carboxylique.

11. Composé qui est un acide (6α,11β,16α,17α)-17-({[(1,1-diméthylpropyl)oxy]carbonyl}oxy]-6,9-difluoro-11-hydroxy-16-méthyl-3-oxoandrosta-1,4-diène-17-carboxylique.

12. Procédé de préparation d'un composé de formule (I) qui comprend la réaction d'un acide carboxylique de formule (II) : 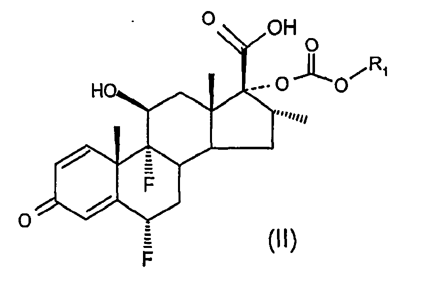 dans laquelle R₁ est tel que défini dans l'une quelconque des revendications 1 à 3 ;
avec un composé de formule L-CH₂-CN, dans laquelle L représente un groupe labile.

13. Procédé de préparation d'un composé de formule (II) 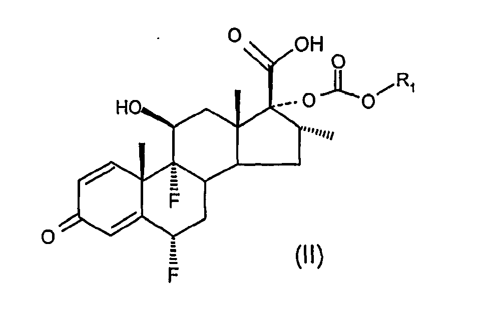 dans laquelle R₁ est tel que défini dans l'une quelconque des revendications 1 à 3, lequel procédé comprend la réaction d'un chloroformiate R₁OCOCl ou d'un anhydre (R₁OCO)₂O avec le dérivé 17α-hydroxyle correspondant de formule (III) :
